Europäisches Patentamt

European Patent Office

Office européen des brevets

(11) Publication number: **0 085 810**
**A2**

# EUROPEAN PATENT APPLICATION

(21) Application number: **82306682.4**

(22) Date of filing: **14.12.82**

(51) Int. Cl.³: **C 12 P 7/08**

(30) Priority: **21.12.81 US 333021**

(43) Date of publication of application:
**17.08.83 Bulletin 83/33**

(84) Designated Contracting States:
**AT BE CH DE FR GB IT LI NL SE**

(71) Applicant: **FUEL IDEAS, INC.**
**Bay 16A 153 Warehouse Mart**
**Chattanooga Tennessee 37421(US)**

(84) Designated Contracting States:
**CH DE FR GB LI SE**

(71) Applicant: **International Cow Power, Inc.**
**Bay 16A 153 Warehouse Mart**
**Chattanooga Tennessee 37421(US)**

(84) Designated Contracting States:
**BE IT NL AT**

(72) Inventor: **Patterson, Roy R.**
**9056 Wooten Road**
**Chattanooga Tennessee(US)**

(72) Inventor: **Morgan, David D.**
**Highway 411 South Box 311**
**Etowah Tennessee(US)**

(74) Representative: **Quest, Barry et al,**
**M'CAW & CO. 41-51 Royal Exchange Cross Street**
**Manchester M2 7BD(GB)**

(54) Process for making alcohol from manure.

(57) A process for making alcohol by fermentation of animal manure mixed with active yeast and water. Fermentation of the mixture is maintained between 60° and 90° F and at a pH in the range of 3.0 to 8.0. After alcohol is obtained in the mash by fermentation it may be distilled and processed.

# PROCESS FOR MAKING ALCOHOL FROM MANURE

## BACKGROUND OF THE INVENTION

Since alcohol is useful as a fuel and as an admixture with hydrocarbon fuels, the diminishing supply of hydrocarbon fuel has led to the development of new techniques for making ethyl alcohol. A conventional method of making alcohol is by breaking down cellulose to simple sugars by means of acids and enzymes. Several methods have recently been devised to break down cellulose by microorganisms at the same time that the end products are fermented by the same or by other microorganisms. Heretofore, however, it has not been known to produce alcohol by fermentation of manure. Although methane gas has been produced from manure by means of anaerobic digestion, the primary use of manure has been as a fertilizer. However, because of its great bulk it is currently of little commercial value.

- 2 -

0085810

## SUMMARY OF THE INVENTION

The present process comprises admixing animal manure with water and an activated yeast and allowing the mixture to ferment at a temperature in the range of between 60° to 80°F while maintaining the pH in a range between 3.0 and 8.0 under anaerobic conditions. The optimal pH value is about 6.5, which appears to be an ideal pH for the manure bacteria and a value at which the yeast can act. Generally, the best results are obtained at a temperature of approximately 72°F. It is theorized that certain bacteria in fresh manure break down the cellulose in the manure when placed in aqueous suspension, and that the sugar formed by this breakdown is utilized by the yeast to form alcohol before methane forming bacteria can produce methane.

## DESCRIPTION OF THE PREFERRED EMBODIMENTS

The basic process comprises essentially the mixing of fresh animal manure with 0.2 to 5.8 percent by weight of Saccharomyces cerevisiae (baker's yeast) or Saccharomyces ellipsoideus (wine or beer yeast) and water, the mass being adjusted to the proper solids content. Thereafter fermentation is allowed to occur under anaerobic conditions, while maintaining the temperature and pH for effective fermentation reactions. The resulting gas is allowed to escape and fermentation continues until completion. The wine yeast provides a greater alcohol yield but requires a relatively longer period in which to complete fermentation, e.g. 20 to 30 days. Baker's yeast or activated dry yeast on the other hand is faster, in the order of 3 to 5 days, but gives a lower alcohol yield. Thus, the best results are obtained by using a combination of these types, but preferably a reseeding culture from a preceeding production run, is used, in which case the alcohol yield is increased substantially. Moreover, the addition of sugar, from sugar bearing waste products, may be added to the mash to speed up the process.

Three basic types of experiments were conducted. In each the waste manure product was weighed in a container with the dry solids being adjusted to approximately 2 to 10% of the total weight. About 50% of the water then was added to reduce viscosity for the weighed material. In those experiments utilizing baker's yeast a small amount of water was heated to 100 to 120°F. and to this the yeast was added along with a small amount of sugar to activate the yeast. In those experiments utilizing wine yeast, the yeast was put on top of the mixture until it began foaming. In each case once reaction began the remainder of the water together with the activated yeast was mixed into the mass and allowed to react until gas production ceased. In the case of baker's yeast this time varied between 2 and 5 days, while in the case of wine yeast the time varied between 20 and 30 days. The fermented mixture was collected and thereafter distilled to determine the alcohol yield by the iodoform test for alcohols, specific gravity and gas chromategraphic analysis. The yield of alcohol per 100 pound animal was then determined from the manure yields disclosed in tables in the book "Methane Generation From Human, Animal, and Alcohol" published by the Fuels Information Center, 1617 Cole Blvd., Golden, Colorado, 80401 (Library of Congress Cat. No. 77-92794). For example, the average daily values of wet weight of manure per 1,000 pound cow there listed was 80.1 pounds at 7.98% dry solids.

<div align="center">EXAMPLE I</div>

Approximately 120 grams of wet waste was mixed with 0.25 grams (dry weight) of wine yeast and 800 grams of water. The solid waste was adjusted by the addition of water to approximately 2% of the total and the yeast was approximately 0.2% of the wet waste. The final total solids of fermented waste varied somewhat due to differences in the total solids of waste material used. The starting temperature varied between 140°F. to 176°F., and the average initial pH was approximately 7.0. Fermentation was maintained at a temperature of between 60° and 90°F. If the pH value dropped below 5.5 it was adjusted back to 7.5 ± 0.5 after 6 days the average pH value was approximately 5.5. The results of these and the following tests are enumerated in the Summary Chart hereinafter.

<u>EXAMPLE II</u>

In these tests 1800 grams of wet waste was mixed with 14 grams of baker's yeast, activated as indicated above, and 2,000 grams of water. The constituants represented 0.77% of yeast to that of wet waste, with the final solids adjusted to approximately 7% depending on the total solids of starting waste. Again the starting temperature was between 140° to 176°F. with an initial pH of 7.0. Fermentation occured while temperature was maintained between 60° and 90°F. and the pH value was adjusted as in experiment I.

## EXAMPLE III

In these experiments 3,000 grams of wet waste were mixed with 200 grams of water, 49 grams of baker's yeast, 10 grams of sugar and 10 grams of a reseeding yeast culture saved from previous experiments. The sugar represents 0.33% of the wet waste and the yeast 1.60% of the wet waste. The final total solids of fermented waste varied between 9.6% to 17.6% of the total solids due to differences in the total solids of waste products in the mixture.

The above three examples represent the most effective percentages of the constituant mass providing the greatest yield of alcohol. Other experiments were conducted during which it was concluded that alcohol production could be obtained from 100 pounds of manure when mixed with between 10 and 80 gallons of water, and between 0.2 and 5.8 pounds of yeast. Although the pH of the mixture was effective in the range between 3.0 and 8.0 the optimum pH was 6.5.

A Summary Chart indicating the results of the above three examples for various animals follows:

## SUMMARY CHART

| Waste From | Method | Alcohol Yield | Alcohol in Gallons per 100 lbs of Wet Waste | Gallons of 200 proof Alcohol per 1,000 lb (Body Weight) Animal |
|---|---|---|---|---|
| Dairy Cows | I | 35.9ml | 1.7gal | 1.3gal |
| | II | 381ml | 1.5gal | 1.1gal |
| | III | 990ml | 3.9gal | 3.1gal |
| Beef Cows | I | 37.6ml | 1.8gal | 1.4gal |
| | II | 420ml | 1.65gal | 1.2gal |
| | III | 1,000ml | 3.94gal | 3.13gal |
| Horses | I | 21.7ml | 1.0gal | 0.76gal |
| | II | 347ml | 1.4gal | 1.0gal |
| | III | 727ml | 2.9gal | 1.0gal |
| Pigs | I | 23.5ml | 1.1gal | 0.84gal |
| | II | 347ml | 1.4gal | 1.0gal |
| | III | 920ml | 2.7gal | 2.1gal |
| Chickens and Turkeys | I | 17.9ml | 0.8gal | 0.61gal |
| | II | 248ml | 0.97gal | 0.62gal |
| | III | 856ml | 3.37gal | 2.7gal |
| Sheep | I | 24.4ml | 1.6gal | 1.22gal |
| | II | 368ml | 1.44gal | 1.01gal |
| | III | 898ml | 3.53gal | 2.8gal |

A commercial process for the production of alcohol may include the following steps:

### Step 1: Mix Tank

Mix thoroughly the desired amount of water, and new yeast culture, or in normal operation a reseeding culture, with the manure. The pH of the mixture is measured and maintained at approximately 6.5. However, a pH ranging from 3.0 to 8.0 may be used successfully. Ideally the temperature should be maintained in a range between 60° and 80°F. but these limits are not critical and good results were obtained through 90°F. Hot water recycle lines may be used in cold weather to increase the waste temperature by the heat loss into waste through pipes.

Based on using various kinds of manure (all animal excrement, including human excrement) or 1 kind of manure alone, alcohol may be produced with the following variables:

1) Total waste including solids - about 100 pounds.

2) Yeast added 0 between 0.2 and 5.8 pounds of various kinds of yeast which normally produce alcohol. These may be Saccharomyces cervisiae, the common beer yeast fungus, or Saccharomyces ellipsoideus activated dry yeast, the wine yeast, or an equivalent amount of these yeasts in a different form.

3) Water added - 10 to 80 gallons

A monitor for pH and temperature control preferably should be used for waste and recycle (water) lines. Flow control valves may be controlled by the monitor to allow flow through the waste heat water recycle lines.

Heavy settling solids may be transferred to a digester or a mass sludge pit to become food for the digester or to to be recycled to a liquid collector to be used later for alcohol production.

Mixed (liquid) waste will be transferred to a holding tank to be held for normally 3 or more days. Flow control valves with on, off (automatic optional) switch may control transfer of heavy settling solids or mixed liquid waste.

Step 2: Add Sugar -- Optional

The use of sugar may sometimes be desirable to speed up the process or to introduce a new or weak culture. Normal operation should see the mass producing its own sugar (glucose) from the waste. Sugar containing waste material such as sugar beets may be disposed of by using in the process.

### Step 3: Holding Tanks

Each day a different holding tank may be used for that day's accumulated waste. The number of holding tanks needed will be determined by the amount of waste calculated to be available.

Each tank may receive its adjusted mixed liquid waste from Step 1, and a percent of reseeding culture from a liquid collector (Step 4B) or normally from a mass sludge pit (Step 5).

The temperature and pH control will continue as in Step 1, except specific gravity monitoring will be necessary.

The fermentation temperature in the mixed liquid will be regulated by heat loss from the hot water recycle lines with a water temperature of 60° to 140°F. A similar monitor control is used as in Step 1.

The pH control may monitor the pH between 8.0 and 3.0. An alarm may be triggered at pH 4.5 to allow the controller to adjust the pH to 6.5. Fresh waste, reseeding culture or a pH buffer may be used to readjust the pH.

A specific gravity monitor will determine the mixed liquid ready to be distilled. Normally, the mixed liquid may remain in the holding tank until needed for distilling. However, a percent of mixed liquid will be needed in the liquid collector to continue the decomposition of heavier settling solids.

The settling solids in the holding tanks will be transferred to the digester as needed but normally will pass through the mass sludge pit to the liquid collector 4B for further processing.

Step 4A:  Add Yeast Culture

A yeast culture (with Step 2 option) or a reseeding culture may be added to (1) Step 1, waste products; (2) Step 3 (only if needed) holding tanks, mixed liquid; (3) Step 4B, mixed liquid settling solids; (4) When reseeding culture is used from Step 5, a pH adjustment will normally be necessary.

When reseeding culture from Step 5, anaerobic digestion, is used, a retention time of one hour or more after pH adjustment is desirable.  Small amounts of air injected or bubbled through the culture will decrease retention time.

Step 4B:  Liquid Collector: An Aerobic Acclimation of Culture and Waste at pH 7.0

Some settling solids in waste, such as whole kernal corn, even after the first distillation, require further anerobic acclimation.

The settling solids from Steps 1, 3, 5 and 6A may be pumped back to a liquid collector to be adjusted for alcohol production.

The necessary percent mixed liquid from the holding tanks will be added to adjust the conditions in the liquid collector to match those found in the holding tanks. The transition will not be immediate so food for the digester may be constantly be drawn off at the bottom of the liquid collector

Intermittent monitoring of mix liquid pH, temperature and specific gravity to the point required for distillation will be necessary.

The best alcohol producing culture will be developed for reseeding use out of the various mixed liquids in the liquid collector. The lighter mixed liquids in the upper portion of the liquid collector may be aerobic and can be used for reseeding. The lower or heavier portion of the liquid collector will be anaerobic and will be used as food for the digester for reseeding. Various sampling points at different elevations measuring specific gravity will determine the draw off point for the reseeding cultures.

Step 5: Digester and Mass Sludge Pits

The digester's primary function will be to produce methane for process fuel to heat water or operate pumps. The digester will also break down heavier settling solids into a usable mixed liquid for alcohol production. This mixed liquid will be drawn off into the sludge pit and returned to the liquid collector for further processing.

Eventually, a heavy sludge will develop in the bottom of the digester and be drawn off to the sludge pit and pumped through an alcohol still for removal of any remaining alcohol. After passing through the alcohol still, the heavy sludge will return to the sludge pit. Any remaining or decomposable material will be pumped back to the digester.

Step 6A: Alcohol Still

The alcohol still will be partly submerged in a water bath, with the temperature of the water controlled within the range of 90° to 98°C, inside the alcohol still.

The primary mixed liquid from mass sludge pit will be pumped into the alcohol still. The remainder, after distillation of the mixed liquid, will be returned to the mass sludge pit.

Step 6B: Alcohol Still

This still will receive the alcohol from Step 6A and remove the remainder of water to concentrate the alcohol to 195 to 200 proof.

The water bath cooker method used in Step 6A will be the same in Step 6B.

The water removed in this still will be used in the hot water recycle lines.

Step 7: Mass Sludge Pit

The heavy sludge with pathogenic bacteria removed will be excellent to add liquid, lime, nitrogen, phosphate and potassium to bring up to the desired fertilizer strength needed for grassland and other crops.

A higher percentage of total solids with a midrange weight, for instance 2.8 lbs., of baker's processed yeast and 10 gallons of water per 100 lbs. of manure produce the highest percent of alcohol yields in less than three days, at pH 6.5 and at room temperature of 72 degrees F.

Once alcohol is obtained in the mash, it is distilled and processed as is customary in the fermentation alcohol industry. The primary heat source for distillation will be methane, with a solar water heater used as an auxiliary heat source to allow collection of methane on sunny days and to be used for pump operation fuel.

The recycle lines for hot water through each unit will be a by-product from distillation.

This process will ordinarily produce between 1.8 and 2.2 gallons, or even up to 4.4 gallons, of alcohol from the daily manure produced by a 1,000 pound cow.

Numerous alterations of the structure herein disclosed will suggest themselves to those skilled in the art. However, it is to be understood that the present disclosure relates to the preferred embodiment of the invention which is for purposes of illustration only and not to be construed as a limitation of the invention. All such modifications which do not depart from the spirit of the invention are intended to be included within the scope of the appended claims.

Having thus described the nature of the invention, what is claimed herein is:

1. A process for making alcohol from animal manure comprising the steps of:

mixing thoroughly manure, water and yeast in the proportions of 100 pounds of manure, 10 to 80 gallons of water and 0.2 to 5.8 pounds of a yeast selected from baker's yeast, wine yeast and their equivalents, maintaining the pH of the mixture between 3.0 and 8.0, maintaining the temperature between 60 and 90 degrees F., placing the resulting mash under anaerobic conditions while allowing carbon dioxide gas to escape and allowing the mash to ferment until bubbling ceases.

2. The process according to Claim 1, wherein approximately 0.2 pounds of wine yeast is mixed with the manure and water.

3. The process according to Claim 1, wherein approximately 0.77 pounds of baker's yeast is mixed with the manure and water.

4. The process according to Claim 1, wherein glucose sugar is added as an expediter of the process.

5. The process according to Claim 1, wherein alcohol is distilled from the fermented mash.

6. The process according to Claim 5, wherein water is removed from the alcohol in a still to concentrate the alcohol to 195 to 200 proof.